# EUROPEAN PATENT APPLICATION

(11) **EP 2 033 999 A1**
(43) Date of publication of application: **11.03.2009**
(21) Application number: 07743363.9
(22) Date of filing: 15.05.2007
(51) Int. Cl.: C09K 3/00, A01N 43/26, A01P 3/00, A61K 8/86, A61K 31/357, A61L 9/01, A61P 17/00, A61Q 11/00, A61Q 15/00

(54) **OZONIZED SURFACTANT**

(30) Priority: 28.06.2006 JP 2006177750
(71) Applicant: Erc Technology Inc., Kawaguchi-shi, Saitama 332-0035 (JP)
(72) Inventor: YAMAZAKI, Kazutoshi, Annaka-shi, Gunma 379-0109 (JP); TANAKA, Akinori, Tokyo 114-0023 (JP)
(74) Representative: Teipel, Stephan
(86) International application number: PCT/JP2007/059928
(87) International publication number: WO 2008/001553

(57) **Abstract**

Disclosed herein is an ozonized surfactant which has the same level of bactericidal and deodorizing abilities as that of ozonized olive oil and which can be evenly applied or sprayed onto and can permeate any place or environment (a highly hydrophilic or hydrophobic place or environment). The ozonized surfactant can be obtained by adding ozone to a surfactant having at least one olefin-based double bond in a hydrophobic group moiety.

## Description

### TECHNICAL FIELD

The present invention relates to an ozonized surfactant which has bactericidal and deodorizing abilities and which can be evenly applied or sprayed onto and can permeate any place or environment (even a highly hydrophilic or hydrophobic place or environment).

### BACKGROUND ART

Ozonized vegetable oil has an ozonide structure in its molecule, and can be produced by bringing vegetable oil (e.g. , olive oil) into contact with ozone gas. It is known that such ozonized vegetable oil has an antibacterial or oxidizing activity and is therefore effective as an antibacterial agent, a deodorant, or a therapeutic drug for skin diseases such as decubital ulcer and ulcer (see Katsuhiko Nakamuro et al., "Fundamental examination about the use of ozonized vegetable oil in medical and environmental fields", Proceedings of the 8th conference of Japan Research Association for the Medical & Hygienic Use of Ozone, pp. 3 to 8, 2003; and "Latest ozone therapy in Europe", Extra edition of Medical & Hygienic Ozone Research, published by Japan Research Association for the Medical & Hygienic Use of Ozone, No. 2, p. 33, 2002).

However, since vegetable oil is a mixture containing various components, its composition varies depending on growing region, weather conditions, and the like, and therefore it is difficult to produce the ozonized vegetable oil with constant quality.

Further, since natural vegetable oil contains an antioxidant substance, it is expected that the antioxidant substance competes with ozone, which interferes with the production of an ozonide compound (see Mugishima et al., "Yushi · Rou (Fats · Waxes)", Kogyo-Kagaku Zensho (Comprehensive Industrial Chemistry), published by Nikkan Kogyo Shimbun Ltd., vol. 32, pp. 37 to 40, 1967).

Further, since ozonized vegetable oil (e.g., ozonized olive oil) is oil, when used for places moistened with water (e.g. , floors and sinks of restaurants and food factories, and bathtubs), biological bodies having hydrophilic surfaces (e.g., fish, vegetables, and fruits), and body parts of mammals (e.g. , the oral cavity, the vagina and a skin wound site), the ozonized vegetable oil is repelled and therefore cannot be evenly applied or sprayed onto the objects. This is a problem in practical use.

Meanwhile, ozone water is also used for treatment such as sterilization, deodorizing, and bleaching. However, there are problems that stably preserving ozone water for a long period of time is difficult and that maintaining an effective ozone concentration in ozone water for such treatment for a long period of time is also difficult. In order to overcome these difficulties, a bactericidal detergent composition containing ozone and a surfactant has been proposed (see Japanese Unexamined Patent Publication No. 06-313194).

However, all the surfactants used for the bactericidal detergent composition (e.g., linear lauryl benzene sulfonic acid, sodium lauryl ether carboxylate, and sodium dioctyl sulfosuccinate) have a saturated hydrocarbon group as a hydrophobic group, and surfactants having an unsaturated aliphatic hydrocarbon group as a hydrophobic group are not disclosed at all. In the case where the surfactant having a saturated aliphatic hydrocarbon group as a hydrophobic group was used, the ozone concentration decreased to about 1/2 at room temperature in 20 minutes. As is clear from the result, attenuation of ozone concentration of the bactericidal detergent composition was at the same level as that of water containing dissolved ozone. This means that the surfactant and ozone water were in a physically-mixed state in the bactericidal detergent composition. The duration of effective ozone concentration was not sufficient, and there still was room for improvement.

Although so-called cationic surfactants classified as invert soap or amphoteric soap have already been used as surfactants having surfactant function and antibacterial and bactericidal activities, the antibacterial and bactericidal activities of these cationic surfactants against filamentous fungi are weaker than those against common bacteria.

### DISCLOSURE OF THE INVENTION

### PROBLEM TO BE SOLVED BY THE INVENTION

An object of the present invention is to provide an ozonized surfactant which can maintain the same level of bactericidal and deodorizing abilities as that of ozonized olive oil for a long period of time and which can be evenly applied or sprayed onto and can permeate any place including a highly hydrophilic or hydrophobic place (or environment).

### MEANS FOR SOLVING THE PROBLEM

The present inventors have intensively studied to achieve the above object, and as a result have found that an ozonized surfactant obtained by adding ozone to a surfactant having at least one olefin-based double bond in a hydrophobic group moiety is superior to one obtained by allowing ozone water to act on a surfactant not having any olefin-based double bond, and has the same level of bactericidal and deodorizing abilities as that of ozonized olive oil. In addition, the present inventors have also found that the ozonized surfactant according to the present invention can be evenly applied or sprayed onto and can permeate any place. Based on these findings, the present inventors have further studied and finally completed the present invention.

Namely, the present invention relates to:
(1) an ozonized surfactant obtained by adding ozone to a surfactant having at least one olefin-based double bond in a hydrophobic group moiety;
(2) the ozonized surfactant according to the above (1), wherein the at least one olefin-based double bond present in a hydrophobic group moiety of the surfactant is converted into an ozonide structure represented by the following formula (I): 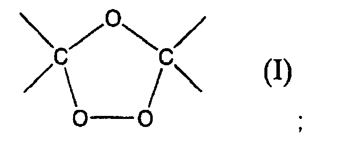
(3) the ozonized surfactant according to the above (1), wherein the surfactant having at least one olefin-based double bond in a hydrophobic group moiety is a surfactant selected from the group consisting of a nonionic surfactant having at least one olefin-based double bond in a hydrophobic group moiety, an anionic surfactant having at least one olefin-based double bond in a hydrophobic group moiety, and a cationic surfactant having at least one olefin-based double bond in a hydrophobic group moiety;
(4) the ozonized surfactant according to the above (3), wherein the nonionic surfactant having at least one olefin-based double bond in a hydrophobic group moiety is a nonionic surfactant selected from glycerol unsaturated fatty acid esters, polyglycerol unsaturated fatty acid esters, propylene glycol unsaturated fatty acid esters, sorbitan unsaturated fatty acid esters, polyoxyethylene sorbitan unsaturated fatty acid esters, polyoxyethyelene unsaturated fatty acid esters, mannitol unsaturated fatty acid esters, pentaerythritol unsaturated fatty acid esters, sucrose unsaturated fatty acid esters, polyethylene glycol unsaturated alkyl ethers, and polypropylene glycol unsaturated alkyl ethers;
(5) the ozonized surfactant according to the above (3), wherein the anionic surfactant having at least one olefin-based double bond in a hydrophobic group moiety is an anionic surfactant selected from unsaturated fatty acid salts, unsaturated aliphatic alcohol sulfate esters, α-sulfo unsaturated fatty acid esters, olefin sulfates, and olefin sulfonates;
(6) the ozonized surfactant according to the above (3), wherein the cationic surfactant having at least one olefin-based double bond in a hydrophobic group moiety is a cationic surfactant selected from unsaturated aliphatic hydrocarbon group-monosubstituted trimethyl ammonium salts, unsaturated aliphatic hydrocarbon group-disubstituted dimethyl ammonium salts, unsaturated aliphatic hydrocarbon group-monosubstituted dimethyl benzyl ammonium salts, unsaturated aliphatic hydrocarbon group-monosubstituted amine organic acid salts, unsaturated aliphatic hydrocarbon group-disubstituted amine organic acid salts, and N-methyl bishydroxyethylamine unsaturated fatty acid ester hydrochlorides;
(7) the ozonized surfactant according to the above (1), wherein the surfactant having at least one olefin-based double bond in a hydrophobic group moiety is a polyethylene glycol unsaturated alkyl ether;
(8) the ozonized surfactant according to the above (1), wherein the surfactant having at least one olefin-based double bond in a hydrophobic group moiety is a polyoxyethylene sorbitan unsaturated fatty acid ester;
(9) the ozonized surfactant according to any one of the above (4) to (8), wherein the unsaturated fatty acid has 8 to 24 carbon atoms;
(10) the ozonized surfactant according to the above (5) or (6), wherein the unsaturated aliphatic hydrocarbon group has 8 to 24 carbon atoms;
(11) the ozonized surfactant according to the above (8), wherein the polyoxyethylene sorbitan unsaturated fatty acid ester is polyoxyethylene sorbitan oleic acid ester;
(12) a pharmaceutical drug which contains the ozonized surfactant according to any one of the above (1) to (11);
(13) a bactericide which contains the ozonized surfactant according to any one of the above (1) to (11);
(14) a disinfectant which contains the ozonized surfactant according to any one of the above (1) to (11);
(15) a deodorant which contains the ozonized surfactant according to any one of the above (1) to (11);
(16) a drug for athlete's foot treatment which contains the ozonized surfactant according to any one of the above (1) to (11);
(17) an antifungal agent which contains the ozonized surfactant according to any one of the above (1) to (11); and
(18) a drug for treatment of decubital ulcers which contains the ozonized surfactant according to any one of the above (1) to (11).

### EFFECT OF THE INVENTION

The ozonized surfactant according to the present invention has the same level of bactericidal and deodorizing abilities as that of ozonized olive oil conventionally used for sterilization and deodorization, and can be evenly applied or sprayed onto and can permeate any place including a highly hydrophilic or hydrophobic place (or environment).

Further, the ozonized surfactant according to the present invention has a bactericidal ability on bacteria and fungi, and particularly has a strong bactericidal ability specifically on filamentous fungi such as Trichophyton and Cladosporium species. Therefore, the ozonized surfactant according to the present invention can be used as an antibacterial agent or a bactericide.

Particularly, the ozonized surfactant according to the present invention is useful as a drug for athlete's foot treatment. The ozonized surfactant has an effect on Trichophyton species present under a stratum corneum of the skin or in the nail, which cannot be achieved by conventional drugs for athlete's foot treatment, due to its own permeation function and wetting function.

Further, the ozonized surfactant according to the present invention has an advantage that it can be easily applied as an antifungal agent onto various places such as highly-hydrophobic vinyl chloride-based wall paper and highly-hydrophilic surfaces of mortar-based sinks and floors due to its wetting function.

Further, the ozonized surfactant according to the present invention has an advantage that it can be easily used for antibacterial treatment of various goods such as socks, shoes, fabrics, mats, and wet tissue paper.

### BRIEF DESCRIPTION OF THE DRAWINGS

Fig. 1 is a flow chart showing a process of synthesizing an ozonized surfactant.
Fig. 2 is a graph showing a temporal change in the concentration of ozone in various surfactant solutions during reaction between a surfactant and ozone.

### REFERENCE SIGNS LIST

1. Ozonizer
2. Ozone decomposing tower
3. Dissolving hollow fiber membrane
4. Module outer case
5. Ozone concentration meter
6. Cyclic reaction tank
7. Circulating pump
8. Ozone gas flow path
9. Surfactant solution flow path

### BEST MODE FOR CARRYING OUT THE INVENTION

An ozonized surfactant according to the present invention can be obtained by adding ozone to a surfactant having at least one olefin-based double bond in a hydrophobic group moiety. The ozonized surfactant is a surfactant where the at least one olefin-based double bond present in a hydrophobic group moiety is converted into an ozonide structure represented by the following formula (I):

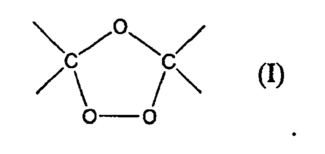

The ozonized surfactant according to the present invention can be obtained by allowing ozone gas to act on a surfactant having at least one olefin-based double bond in a hydrophobic group moiety.

As a raw material surfactant having at least one olefin-based double bond in a hydrophobic group moiety, a surfactant having a resonating double bond (e.g., a double bond in benzene) in its molecule should be avoided because ozone acting on the site of a resonating double bond impairs previously-designed properties of the surfactant. The surfactant to be used as a raw material preferably can be dissolved or dispersed in water.

Such a surfactant to be used as a raw material is not particularly limited, and representative examples include the following surfactants.
(A) Nonionic surfactants
   1 glycerol unsaturated fatty acid ester
      structural formula: R-COO-CH₂CH(OH)CH₂OH
   2 polyglycerol unsaturated fatty acid ester
      structural formula: R-COO-(CH₂CH(OH)CH₂O)n-H
   3 propylene glycol unsaturated fatty acid ester
      structural formula: RCOO-(CH₂CH(CH₃)O-)n-H
   4 sorbitan unsaturated fatty acid ester structural formula: R-COO-[sorbitan]
   5 polyoxyethylene sorbitan unsaturated fatty acid ester
      structural formula (monoester): R-COO-[sorbitan]-((CH₂CH₂O)n-H)m
   6 polyoxyethylene unsaturated fatty acid ester
      structural formula: R-COO-(CH₂CH₂O)n-H
   7 mannitol unsaturated fatty acid ester
      structural formula (monoester): R-COO-[mannitol]
   8 pentaerythritol unsaturated fatty acid ester
      structural formula (monoester): R-COO-[pentaerythritol]
   9 sucrose unsaturated fatty acid ester
      structural formula: R-COO-[sucrose]
   10 polyethylene glycol unsaturated alkyl ether
      structural formula: R¹-O-(CH₂CH₂O)n-H
   11 polypropylene glycol unsaturated alkyl ether
      structural formula: R¹-O-(CH₂CH(CH₃)O-)n-H
   12 others
      The above-mentioned nonionic surfactant having a polysaccharide or a polyhydric alcohol as a structural component (e.g., the above-mentioned 1, 2, 4, 7, 8, or 9) may be a derivative obtained by further addition of polyethylene glycol or polypropylene glycol.
(B) Anionic surfactants
   1 unsaturated fatty acid salt
      structural formula: R-COOM
   2 unsaturated aliphatic alcohol sulfate ester(higher alcohol sulfate) structural formula: R²-O-SO₃M
   3 α-sulfo unsaturated fatty acid ester
      structural formula: R¹-CH(-SO₃M)-COOR^{a}
   4 olefin sulfonate
      structural formula: R-SO₃M
   5 olefin sulfate
      structural formula: R-SO₄M
(C) Cationic surfactants
   1 unsaturated aliphatic hydrocarbon group-monosubstituted trimethyl ammonium salt
      structural formula: R²-N⁺(CH₃)₃Cl⁻
   2 unsaturated aliphatic hydrocarbon group-disubstituted dimethyl ammonium salt
      structural formula: (R²)₂N⁺(CH₃)₂Cl⁻
   3 unsaturated aliphatic hydrocarbon group-monosubstituted dimethyl benzyl ammonium salt
      structural formula: R²-N⁺(CH₃)₂(-CH₂-C₆H₅)Cl⁻
   4 unsaturated aliphatic hydrocarbon group-monosubstituted amine organic acid salt
      structural formula: [R²-NH₃]⁺_{·}[R^{b}COO]⁻
   5 unsaturated aliphatic hydrocarbon group-disubstituted amine organic acid salt
      structural formula: [R₂NH₂]⁺·[R^{b}COO]⁻
   6 N-methylbishydroxyethylamine unsaturated fatty acid ester hydrochloride
      structural formula: (R-COO-CH₂CH₂)₂NCH₃·HCl

In the above structural formulas, R represents a residue obtained by removing a carboxyl group from an unsaturated fatty acid (R-COOH), R¹ and R² each represent an unsaturated aliphatic hydrocarbon group, R^{a} represents a lower alkyl group, R^{b} represents a residue obtained by removing a carboxyl group from an organic acid (R^{b}-COOH), and M represents an alkali metal, an alkaline-earth metal, monoethanolamine, or diethanolamine. Although the above representative examples of esters are structural formulas of monoesters, the esters may be polyesters such as diesters and triesters.

Here, examples of the unsaturated fatty acid include, but are not limited to, unsaturated fatty acids having 8 to 24 carbon atoms and 1 to 6 olefin-based double bonds. Specific examples of such unsaturated fatty acids are as follows.

Specific examples of an unsaturated fatty acid having one olefin-based double bond (a monounsaturated fatty acid) include crotonic acid, myristoleic acid, palmitoleic acid, oleic acid, elaidic acid, vaccenic acid, gadoleic acid, erucic acid, and nervonic acid.

Specific examples of an unsaturated fatty acid having two olefin-based double bonds (a diunsaturated fatty acid) include linoleic acid.

Specific examples of an unsaturated fatty acid having three olefin-based double bonds (a triunsaturated fatty acid) include linolenic acid and eleostearic acid.

Specific examples of an unsaturated fatty acid having four olefin-based double bonds (a tetraunsaturated fatty acid) include stearidonic acid, arachidonic acid, eicosapentaenoic acid, and clupanodonic acid.

Specific examples of an unsaturated fatty acid having six olefin-based double bonds (a hexaunsaturated fatty acid) include docosahexaenoic acid.

Examples of the unsaturated aliphatic hydrocarbon group represented by R¹ or R² include groups having 8 to 24 carbon atoms and 1 to 6 olefin-based double bonds.

Examples of the lower alkyl group represented by R^{a} include lower alkyl groups having 1 to 5 carbon atoms, and specific examples of such a lower alkyl group include a methyl group and an ethyl group.

Examples of the alkali metal represented by M include sodium, potassium, and lithium, and a preferred example of the alkaline-earth metal represented by M includes calcium.

The surfactant having at least one olefin-based double bond in a hydrophobic group moiety may be a commercially-available one or one synthesized by a method known per se.

Examples of a method for producing an ozonized surfactant according to the present invention using the above-described surfactant having at least one olefin-based double bond in a hydrophobic group moiety (hereinafter, referred to as a "raw material surfactant" or simply referred to as a "surfactant") include a method in which an aqueous solution of the surfactant is subjected to ozone gas bubbling. When diluted with water or the like, a surfactant becomes difficult to handle due to frothing. In order to easily produce an ozonized surfactant, an apparatus equipped with an ozone dissolving module with a hollow tubular ozone gas-permeable membrane in its outer case. Such an apparatus is described in, for example, Japanese Unexamined Patent Publication No. 2005-161163. The ozone gas-permeable membrane is preferably a non-porous membrane. Preferred examples of the material of the ozone gas-permeable membrane include fluorine-based resins and silicon-based resins described in the Japanese Unexamined Patent Publication.

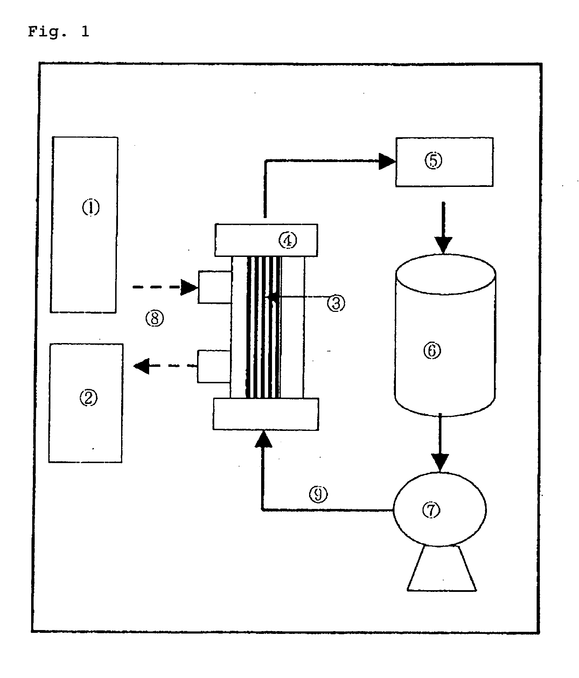

A process flow for synthesizing an ozonized surfactant is concretely shown in Fig. 1. An aqueous solution of a surfactant to be treated is charged into a cyclic reaction tank 6, and then supplied through a flow path 9 into an ozone gas-permeable hollow fiber membrane 3 in a module outer case 4 by a circulating pump 7. The surfactant is ozonized in the hollow fiber membrane, and aqueous solution containing the ozonized surfactant is sent back to the cyclic reaction tank 6 via an ozone concentration meter 5.

Meanwhile, ozone gas is produced from oxygen supplied from an oxygen cylinder to an ozonizer 1, is supplied into an ozone dissolving module through a flow path 8 so as to penetrate the hollow fiber membrane 3, is dissolved in the aqueous surfactant solution in the hollow fiber membrane, and then ozonizes the surfactant to provide an ozonized surfactant. The reaction of forming an ozonide is partially represented by the following formula.

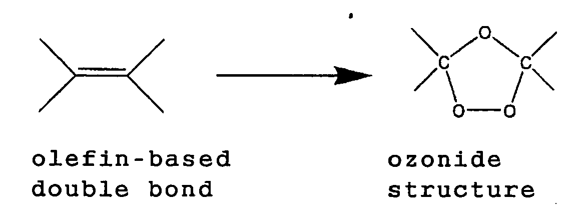

After deaerating the obtained aqueous ozonized surfactant solution to remove dissolved ozone gas by helium purging, the oxidizing property of the aqueous ozonized surfactant solution can be determined by the iodine titration method (see Katsuhiko Nakamuro et al., "Fundamental examination about the use of ozonized vegetable oil in medical and environmental fields", Proceedings of the 8th conference of Japan Research Association for the Medical & Hygienic Use of Ozone, pp. 3 to 8, 2003). Whether or not the aqueous ozonized surfactant solution maintains a function as a surfactant can be evaluated by checking whether or not frothing, which is a characteristic of surfactants, occurs when the solution is agitated or vibrated.

The ozonized surfactant according to the present invention has excellent oxidizing property, and therefore can be used for, for example, sterilizing, disinfecting, or deodorizing places moistened with water (e. g. , floors and sinks of restaurants and food factories, and bathtubs). Further, the ozonized surfactant according to the present invention can also be used for, for example, sterilizing or deodorizing biological bodies having hydrophilic surfaces (e.g., fish, meat, vegetables, and fruits) and parts of the human body (e.g., the oral cavity, the vagina, a decubital ulcer site and a wound site).

For these purposes, the ozonized surfactant according to the present invention is used it may be used as it is without any processing or processed into various drug formulations with appropriate carriers, such as general drugs for external use (e.g., ointments, tapes, gel patches, creams, lotions, liniments, liquid drugs, and aerosols), drugs for external use to be administered into the rectum, vagina, or urethra (e.g., suppositories, enema agents, and impregnation agents), eye-drops, nasal drops, eardrops, injections, and drugs for the mouth. These drugs can be produced by methods well known per se.

For example, in a case where the ozonized surfactant according to the present invention is processed into semisolid drugs such as ointments and creams, the amount of the ozonized surfactant contained in a semisolid drug is, for example, about 0.01 to 50% (W/W) , preferably about 0.1 to 10% (W/W) with respect to the total amount of the semisolid drug. In a case where the ozonized surfactant according to the present invention is processed into gelled drugs such as gel patches, the amount of the ozonized surfactant contained in a gelled drug is, for example, about 0.01 to 50% (W/W), preferably about 0.1 to 10% (W/W) with respect to the total amount of the gelled drug. In a case where the ozonized surfactant according to the present invention is processed into liquid drugs such as injections, eye-drops, and intravenous fluids, the amount of the ozonized surfactant contained in a liquid drug is, for example, about 0.001 to 10% (W/W), preferably about 0.01 to 1.0% (W/W) with respect to the total amount of the liquid drug.

In producing these drugs, carriers or additives suitable for the formulation can be used. Examples of such carriers or additives include: excipients (e.g., sodium polyacrylate, calcium polyacrylate, carboxymethyl cellulose, lactose, dextrin, corn starch, crystalline cellulose, sucrose, sodium chloride, glucose, urea, starch, calcium carbonate, kaoline, silicic acid, and potassium phosphate); lubricants (e.g., magnesium stearate, sucrose fatty acid esters, glycerol fatty acid esters, purified talc, and polyethylene glycol); disintegrants (e.g., carboxymethylcellulose calcium, anhydrous calcium hydrogen phosphate, carboxymethylcellulose sodium, low-substituted hydroxypropylcellulose , dry starch, sodium alginate, agar powder, laminaran powder, sodium hydrogen carbonate, and calcium carbonate); binders (e.g., hydroxypropylcellulose, gum arabic solution, water, ethanol, propanol, simple syrup, dextrose in water, starch solution, gelatin solution, carboxymethyl cellulose, methylcellulose, and polyvinyl pyrrolidone); solubilizing aids(e.g., gum arabic and polysorbate 80); absorption promoters (e.g., quaternary ammonium bases and sodium lauryl sulfate); buffering agents (e.g., phosphate buffer, acetic acid buffer, boric acid buffer, carbonic acid buffer, citric acid buffer, tris buffer, glutamic acid buffer, and epsilon aminocaproic acid buffer); preservatives (e.g., methyl parahydroxybenzoate, ethyl parahydroxybenzoate, propyl parahydroxybenzoate, butyl parahydroxybenzoate, chlorobutanol, benzyl alcohol, benzalkonium chloride, sodium dehydroacetate, and edetate sodium); thickeners (e.g., propylene glycol, glycerol, hydroxyethyl cellulose, hydroxypropyl cellulose, polyvinyl alcohol, and polyethylene glycol); stabilizers (e.g., sodium hydrogen sulfite, sodium thiosulfate, edetate sodium, sodium citrate, ascorbic acid, and dibutylhydroxytoluene); and pH regulators (e.g., hydrochloric acid, sodium hydroxide, phosphoric acid, and acetic acid).

In a case where the ozonized surfactant according to the present invention is used for the human body as a pharmaceutical drug such as a drug for wound treatment, a drug for treatment of decubital ulcers, a bactericide, a deodorant, or a drug for athlete's foot treatment, the dosage of such a drug differs depending on target object, target site, conditions of target site, dosage form, etc. For example, in the case of a semisolid drug, the dosage of the ozonized surfactant as an active ingredient is about 1 to 100 mg, preferably about 1 to 10 mg per about 10 cm² of target site area per day. In the case of a gelled drug, the dosage of the ozonized surfactant as an active ingredient is about 1 to 100 mg, preferably about 1 to 10 mg per about 10 cm² of target site area per day. In the case of a liquid drug, the dosage of the ozonized surfactant as an active ingredient is about 1 to 100 mg, preferably about 1 to 10 mg per about 10 cm² of target site area per day.

Further, in a case where the ozonized surfactant according to the present invention is used as a bactericide, disinfectant, or deodorant for an object other than the human body, the amount of the ozonized surfactant to be used is appropriately determined depending on target site, target site area, and the like.

Specific examples of a drug containing the ozonized surfactant according to the present invention include deodorant toothpastes, deodorants for eliminating breath odor, body odor, or foot odor, bactericides for sterilizing the sites of diseases such as allergy, gum disease, athlete's foot, and decubital ulcers, and bactericides for keeping fish, meat, vegetables, and fruits fresh.

A deodorant spray containing the ozonized surfactant according to the present invention may contain a propellant such as dichlorodifluoromethane to pressurize the deodorant for spraying.

Further, a deodorant toothpaste containing the ozonized surfactant according to the present invention may contain silica, hydroxyapatite etc. as a filler.

### EXAMPLES

Hereinbelow, the present invention will be described in detail with reference to examples, but is not limited to these examples.

Examples 1 to 4 as production examples of the ozonized surfactant according to the present invention, Comparative Examples 1 to 4 corresponding to Examples 1 to 4, and Test Examples 1 to 3 will be described below.

### (Example 1)

An ozonized surfactant was synthesized according to a process flow shown in Fig. 1. More specifically, 2 L of a 0.1% aqueous solution of polyethylene glycol 20 sorbitan monooleate (Tween 80 manufactured by Wako Pure Chemical Industries, Ltd.) as a nonionic surfactant was charged into a cyclic reaction tank 6 made of PFA (a tetrafluoroethylene-perfluoroalkyl vinyl ether copolymer), supplied by a pump 7 into a dissolving hollow fiber membrane 3 in a module outer case 4, and sent back to the cyclic reaction tank 6 through an ozone detector 5 (ozone concentration meter EL-600 manufactured by EBARA Corporation).

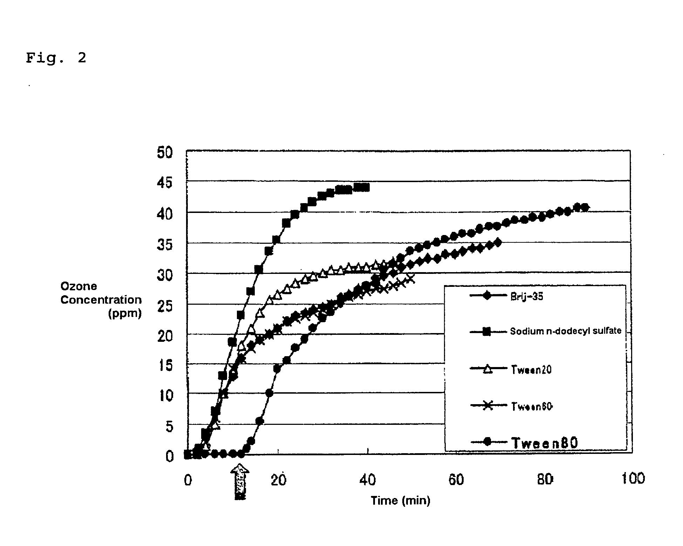

Meanwhile, ozone gas was supplied from an oxygen cylinder into an ozonizer (ozonizer GR-RB manufactured by Sumitomo Precision Products Co. , Ltd.) at an oxygen flow rate of 0.3 L/min to ozonize the oxygen to produce ozone gas. Then, the ozone gas was dissolved in the above-described aqueous solution in a dissolving module (SEK model manufactured by ERC Technology), and the aqueous solution was circulated at a flow rate of 1 L/min. Continuous measurement of the concentration of ozone in the aqueous solution indicated that, unlike a case where pure water was used, the ozone concentration did not increase at all in the early stage, and gradually increased after 14 minutes had elapsed, which confirmed that the reaction of ozone with unsaturated groups had been completed. Further, in 30 minutes, the concentration increased to 25 ppm (see Fig. 2). After deaerating the aqueous solution that had undergone about 30-minute ozone treatment to remove dissolved ozone gas by helium purging, oxidizing property of the aqueous solution was determined by the iodine titration method. As a result, the aqueous solution had oxidizing property, and frothing, which is a characteristic of surfactants, was observed. The results confirmed that ozonized Tween 80 had been produced. Further, a temporal change in the oxidizing property of the aqueous solution was determined by the iodine titration method at 25°C for 20 days. As a result, the percentage of oxidizing property measured after 20 days to that measured just after preparation was 109%. This indicates that the oxidizing property of the aqueous solution was not attenuated at all.

### (Example 2)

Ozone treatment was carried out using the same system under the same conditions as in Example 1 except that instead of Tween 80, polyethylene glycol 20 oleyl ether (manufactured by Wako Pure Chemical Industries, Ltd.) was used. The concentration of ozone in the aqueous solution gradually increased after 16 minutes had elapsed, which confirmed that the reaction of ozone with unsaturated groups had been completed. Further, in 30 minutes, the concentration increased to 30 ppm. After deaerating the aqueous solution that had undergone about 30-minute ozone treatment to remove dissolved ozone gas by helium purging, oxidizing property of the aqueous solution was determined by the iodine titration method. As a result, the aqueous solution had oxidizing property, and frothing, which is a characteristic of surfactants, was observed. The results confirmed that ozonized polyethylene glycol 20 oleyl ether had been produced.

### (Example 3)

Ozone treatment was carried out using the same system under the same conditions as in Example 1 except that instead of Tween 80, polyethylene glycol 20 sorbitan trioleate (Tween 85 manufactured by Wako Pure Chemical Industries, Ltd.) was used as a nonionic surfactant. The concentration of ozone in the aqueous solution gradually increased after 22 minutes had elapsed, which confirmed that the reaction of ozone with unsaturated groups had been completed. After deaerating the aqueous solution that had undergone about 30-minute ozone treatment to remove dissolved ozone gas by helium purging, oxidizing property of the aqueous solution was determined by the iodine titration method. As a result, the aqueous solution had oxidizing property, and frothing, which is a characteristic of surfactants, was observed. The results confirmed that ozonized Tween 85 had been produced.

### (Example 4)

Polyethylene glycol 10 oleyl ether (manufactured by Wako Pure Chemical Industries, Ltd.) was dissolved in pure water to prepare a 10 wt% aqueous surfactant solution, and this aqueous solution was used as a raw material to synthesize an ozonized surfactant. An ozonized surfactant was synthesized by treating the aqueous solution according to a process flow shown in Fig. 1. More specifically, 500 mL of the aqueous solution was charged into the cyclic reaction tank 6, supplied by the pump 7 into the dissolving hollow fiber membrane 3 in the module outer case 4, and sent back to the cyclic reaction tank 6 through the ozone detector 5 (ozone concentration meter EL-600 manufactured by EBARA Corporation).

Meanwhile, ozone gas was supplied from an oxygen cylinder into an ozonizer (ozonizer GR-RB manufactured by Sumitomo Precision Products Co., Ltd.) at an oxygen flow rate of 0.3 L/min to ozonize the oxygen to produce ozone gas. The ozone gas was circulated at a liquid supply rate of 1 L/min. Then, the ozone gas was dissolved in the above-described aqueous solution in a dissolving module (SEK model manufactured by ERC Technology), and the aqueous solution was circulated at a flow rate of 1 L/min and the concentration of ozone in the aqueous solution was continuously monitored. A point at which the concentration of ozone in the aqueous solution treated with ozone increased was defined as the end point of reaction. In this way, an ozonized surfactant was synthesized. Hereinafter, the ozonized surfactant solution prepared in Example 4 will be referred to as "solution A".

The solution A was diluted to obtain a 1% aqueous solution, and a temporal change in the oxidizing property of the aqueous solution was determined by the iodine titration method at 25°C for 200 days. As a result, the percentageo of oxidizing property measured after 200 days to that measured just after preparation was 101%. This indicates that the oxidizing property of the aqueous solution was not attenuated at all.

### (Comparative Example 1)

Ozone treatment was carried out using the same system under the same conditions as in Example 1 except that instead of Tween 80, polyethylene glycol 20 sorbitan monostearate (Tween 60 manufactured by Wako Pure Chemical Industries, Ltd.) was used as a nonionic surfactant. The concentration of ozone in the aqueous solution gradually increased from the beginning of ozone treatment. The result confirmed that the reaction between ozone and the surfactant did not occur (see Fig. 2). In 30 minutes from the beginning of ozone treatment, the concentration of ozone in the aqueous solution increased to 25 mg/L. After deaerating the aqueous solution that had undergone about 30-minute ozone treatment to remove dissolved ozone gas by helium purging, the oxidizing property of the aqueous solution was determined by the iodine titration method. As a result, the aqueous solution did not have oxidizing property. However, the aqueous solution exhibited frothing, which confirmed that the surfactant had not been decomposed by ozone.

Thus when a stearyl group was used instead of an oleyl group in Example 1, reaction between ozone and the surfactant did not occur at all.

### (Comparative Example 2)

Ozone treatment was carried out using the same system under the same conditions as in Example 1 except that instead of Tween 80, saturated aliphatic group-containing polyethylene glycol 20 sorbitan monolaurate (Tween 20 manufactured by Wako Pure Chemical Industries, Ltd.), sodium n-dodecyl sulfate, or sodium stearate as a nonionic surfactant was used. In all the three cases, the concentration of ozone in the aqueous solution gradually increased from the beginning of ozone treatment, and in about 30 minutes from the beginning of ozone treatment, the concentration of ozone in the aqueous solution increased to 25 mg/L, 40 mg/L, or 33 mg/L, respectively. The result confirmed that the reaction between ozone and the surfactant did not occur (see Fig. 2). Further, in each case, after deaerating the aqueous solution that had undergone about 30-minute ozone treatment to remove dissolved ozone gas by helium purging, the oxidizing property of the aqueous solution was determined by the iodine titration method. As a result, none of the aqueous solutions had oxidizing property. Thus when a stearyl group was used instead of an oleyl group in Example 1 or a sulfate group or a carboxylate group was used instead of a hydrophilic group, reaction between ozone and the surfactant did not occur at all.

### (Comparative Example 3)

Ozone treatment was carried out using the same system and the same concentration of aqueous surfactant solution under the same conditions as in Example 1 except that instead of Tween 80, n-dodecylbenzenesulfonic acid was used as an anionic surfactant (manufactured by Kanto Chemical Co., Inc.) and the surfactant was adjusted to pH 6.5 with sodium hydroxide. The concentration of ozone in the aqueous solution did not increase (see Fig. 2) even though a hydrophobic group is saturated aliphatic hydrocarbon, and a frothing phenomenon, which is a characteristic of surfactant solutions, was attenuated. The result clearly confirmed that the aromatic ring of the surfactant had been decomposed by ozone.

### (Comparative Example 4)

Ozone treatment was carried out using the same system under the same conditions as in Example 1 except that instead of Tween 80, Brij-35 (C₁₂H₂₅-O-(CH₂CH₂O)₂₃H manufactured by Wako Pure Chemical Industries, Ltd.) was used as a nonionic surfactant. The concentration of ozone in the aqueous solution gradually increased from the beginning of ozone treatment. The result confirmed that the reaction between ozone and the surfactant did not occur (see Fig. 2). In about 30 minutes from the beginning of ozone treatment, the concentration of ozone in the aqueous solution increased to 25 mg/L. After deaerating the aqueous solution that had undergone about 30-minute ozone treatment to remove dissolved ozone gas by helium purging, oxidizing property of the aqueous solution was determined by the iodine titration method. As a result, the aqueous solution did not have oxidizing property. However, the aqueous solution exhibited frothing, which confirmed that the surfactant had not been decomposed by ozone. From the result, it was found that the reaction between ozone and Brij-35 had not occurred at all.

### (Test Example 1)

### Minimum Inhibitory Concentration Test

In order to check the antibacterial and antifungal performance of the ozonized surfactant according to the present invention, bacteriostatic and bactericidal effects of the ozonized surfactant against five kinds of microorganisms were examined. The five kinds of target microorganisms were Staphyrococcus aureus as a prokaryotic gram-positive bacterium, Escherichia coli as a prokaryotic gram-negative bacterium, Saccharomyces cerevisiae and Trichophyton mentagrophytes as fungi which are eukaryotic microorganisms, and Cladosporium cladosporioides. One loopful of each strain was inoculated in a medium optimum for the strain, and cultured under culture conditions optimum for the strain to prepare a cell suspension for inoculation.

The solution A obtained in Example 4 was placed in test tubes, and was then diluted with sterilized water 2-, 4-, 8-, 16-, 32-, 64-, 128-, 256-, and 512-fold to prepare 10 levels of diluted ozonized surfactant solutions. 9 mL of each medium shown in Table 1 was dispensed into L-shaped test tubes for a biophotorecorder, and was then sterilized by steam. Then, 1 mL of each of the 10 levels of diluted ozonized surfactant solutions was added aseptically to the L-shaped test tubes to prepare 10 mL of 5-, 10-, 20-, 40-, 80-, 160-, 320-, 640-, 1280-, and 2560-fold diluted solutions of the solution A (hereinafter, referred to as a "graduated dilution series to be tested"). 0.1 mL of the cell suspension for inoculation was inoculated into the graduated dilution series to be tested (the initial number of cells of each microorganism is shown in Table 1). The L-shaped test tubes into which the cell suspension had been inoculated were set in a biophotorecorder (manufactured by Advantec Toyo Kaisha, Ltd.) and subjected to shaking culture (30 rpm) under conditions shown in Table 1. It is to be noted that the test tubes were sterilized by dry heat (165°C, 1 hour) before use, and the media, sterilized water, and tools were sterilized by steam in an autoclave (121°C, 15 minutes) before use. In the following tests, they were sterilized in the same manner.

**Table 1**

| Microorganisms | Incubation time | Incubation temperature | Initial number of cells | Culture media |
|---|---|---|---|---|
| Staphyrococcus aureus | 24 H | 37°C | 1 × 10⁷/mL | Bouillon liquid medium for sensitivity measurement |
| Escherichia coli | 24 H | 30°C | 1 × 10⁷/mL | Bouillon liquid medium for sensitivity measurement |
| Saccharomyces cerevisiae | 48 H | 28°C | 1 × 10⁶/mL | YM liquid medium |
| Trichophyton mentagrophytes | 96 H | 25°C | 1 × 10⁵/mL | Sabouraud medium |
| Cladosporium cladosporioides | 96 H | 25°C | 1 × 10⁵/mL | Sabouraud medium |

In the dilution series, the minimum concentration of the ozonized surfactant at which turbidity due to microbial growth did not occur within the incubation time was defined as a temporary minimum inhibitory concentration. Another graduated dilution series was prepared between the temporary minimum inhibitory concentration and the concentration of the ozonized surfactant nearest to the temporary minimum inhibitory concentration, at which turbidity occurred. Then, the same operation as described above was again carried out. In the dilution series, the minimum concentration of the ozonized surfactant at which turbidity did not occur was defined as a minimum inhibitory concentration (MIC). It is to be noted that this MIC test against these five kinds of microorganisms was carried out also using, as a control, the surfactant solution not subjected to ozone treatment. The test results are shown in Table 2.

**Table 2**

| Microorganisms | Ozonized surfactant solution minimum inhibitory concentration | Surfactant solution (control) |
|---|---|---|
| Staphyrococcus aureus | 0.11% | > 1% |
| Escherichia coli | 0.25% | > 1% |
| Saccharomyces cerevisiae | 0.02% | > 1% |
| Trichophyton mentagrophytes | 0.03% | > 1% |
| Cladosporium cladosporioides | 0.09% | > 1% |

| | | |
|---|---|---|
| In the table, "%" refers to "% by weight". | | |

As can be seen from Table 2, the 1% surfactant solution (control) not subjected to ozone treatment had no antibacterial and bacteriostatic activities against all the microorganisms, whereas the ozonized surfactant according to the present invention had the effect of suppressing the growth of all the microorganisms.

### (Test Example 2)

### Minimum Bactericidal Concentration Test

0.1 mL of a solution was sampled from each of the L-shaped test tubes of the graduated dilution series to be tested, in which turbidity due to microbial growth did not occur in the MIC test, and was inoculated in an L-shaped test tube containing 10 mL of a medium separately prepared shown in Table 3 and incubated under conditions shown in Table 3. In this case, the ozonized surfactant was diluted 100-fold, and therefore the ozonized surfactant was considered to be ineffective. In the graduated dilution series to be tested, the minimum concentration of the ozonized surfactant at which turbidity did not occur within the incubation time was defined as a minimum bactericidal concentration (MBC). The test results are shown in Table 4.

**Table 3**

| Microorganisms | Incubation time | Incubation temperature | Culture media |
|---|---|---|---|
| Staphyrococcus aureus | 24 H | 37°C | SCDLP bouillon liquid medium |
| Escherichia coli | 24 H | 30°C | SCDLP bouillon liquid medium |
| Saccharomyces cerevisiae | 48 H | 28°C | YM liquid medium |
| Trichophyton mentagrophytes | 96 H | 25°C | Sabouraud liquid medium |
| Cladosporium cladosporioides | 96 H | 25°C | Sabouraud liquid medium |

**Table 4**

| Microorganisms | Ozonized surfactant solution minimum bactericidal concentration |
|---|---|
| Staphyrococcus aureus | 0.11% |
| Escherichia coli | 0.30% |
| Saccharomyces cerevisiae | 0.03% |
| Trichophyton mentagrophytes | 0.03% |
| Cladosporium cladosporioides | 0.18% |

| | |
|---|---|
| In the table, "%" refers to "% by weight". | |

As can be seen from Table 4, the ozonized surfactant according to the present invention had a bactericidal ability against all the microorganisms. Particularly, it was found that the ozonized surfactant according to the present invention had a bactericidal effect on Trichophyton mentagrophytes and Cladosporium cladosporioides even at a low concentration.

### (Test Example 3)

### Surface-active property test

The surface-active properties of ozonized surfactants were determined in the following manner. 1 wt% of aqueous solutions of surfactants (Tween 80, polyethylene glycol 10 oleyl ether, and polyethylene glycol 20 oleyl ether (manufactured by Wako Pure Chemical Industries, Ltd.)) were prepared, and each of the solutions was dropped on a polyvinyl chloride plate to measure a contact angle (θ) using a contact angle meter CA-VP (manufactured by Kyowa Interface Science Co., Ltd.). The same measurement was carried out for the ozonized surfactants obtained in Examples 1, 2 and 4. The test results are shown in Table 5.

**Table 5**

| Surfactants | Contact angle (θ) | |
|---|---|---|
| | Surfactant not subjected to ozone treatment | Surfactant subjected to ozone treatment |
| Tween 80 | 66.5 | 39.3 |
| Polyethylene glycol (10) oleyl ether | 51.9 | 27.8 |
| Polyethylene glycol (20) oleyl ether | 59.4 | 26.7 |

| | | |
|---|---|---|
| In the table, the unit of the contact angles is degree (°). The contact angle of ion-exchange water was 87.3°. | | |

As can be seen from Table 5, the contact angle of each of the aqueous ozonized surfactant solution was smaller than that of ion-exchange water. The result confirmed that all the ozonized surfactants had surface-active ability.

The ozonized surfactant according to the present invention can be used in various forms depending on its application. Specific examples of the application of the ozonized surfactant according to the present invention include bactericidal and disinfectant solutions, gels for external use, breath sprays, and toothpastes, and their compositions are shown below. It is to be noted that "ozonized Tween 80" shown in the following blending examples was prepared by filtering the solution treated with ozone for 30 minutes in Example 1 and drying it.

### (Blending Example 1: solution for external use)

**Table 6**

| Composition | Content (wt%) |
|---|---|
| Ozonized Tween 80 | 10 |
| Purified water | 84 |
| Propylene glycol | 5 |
| Sodium acrylate | 0.5 |
| Carboxymethyl cellulose | 0.5 |
| Total | 100 |

### (Blending Example 2: gel for external use)

**Table 7**

| Composition | Content (wt%) |
|---|---|
| Ozonized Tween 80 | 20 |
| Ethanol | 5 |
| Glycerol | 5 |
| Sodium acrylate | 3 |
| Calcium chloride | 0.01 |
| Purified water | remainder |
| Total | 100 |

### (Blending Example 3: breath spray)

**Table 8**

| Composition | Content (wt%) |
|---|---|
| Ozonized Tween 80 | 0.5 |
| Ethanol | 50 |
| Dichlorodifluoromethane | 49.5 |
| Total | 100 |

### (Blending Example 4: toothpaste)

**Table 9**

| Composition | Content (wt%) |
|---|---|
| Ozonized Tween 80 | 3 |
| Aluminum hydroxide | 45 |
| Sorbitol | 30 |
| Sodium lauryl sulfate | 0.8 |
| Sodium alginate | 0.6 |
| Saccharine sodium | 0.1 |
| Gelatin | 0.2 |
| Lauric diethanolamide | 1.6 |
| Propylene glycol | 5 |
| Perfume | 0.3 |
| Sodium lauroyl sarcosinate | 0.4 |
| Sodium monofluorophosphate | 0.75 |
| Purified water | remainder |
| Total | 100 |

### INDUSTRIAL APPLICABILITY

The ozonized surfactant according to the present invention has the same bactericidal and deodorizing power as that of ozonized olive oil conventionally used for sterilization and deodorizing, and therefore can be used as a bactericide, disinfectant, deodorant, antifungal agent, drug for athlete's foot treatment, and drug for treatment of decubital ulcers.

## Claims

1. An ozonized surfactant obtained by adding ozone to a surfactant having at least one olefin-based double bond in a hydrophobic group moiety.

2. The ozonized surfactant according to claim 1, wherein the at least one olefin-based double bond present in a hydrophobic group moiety of the surfactant is converted into an ozonide structure represented by the following formula (I):

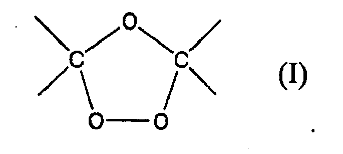

3. The ozonized surfactant according to claim 1, wherein the surfactant having at least one olefin-based double bond in a hydrophobic group moiety is a surfactant selected from the group consisting of a nonionic surfactant having at least one olefin-based double bond in a hydrophobic group moiety, an anionic surfactant having at least one olefin-based double bond in a hydrophobic group moiety, and a cationic surfactant having at least one olefin-based double bond in a hydrophobic group moiety.

4. The ozonized surfactant according to claim 3, wherein the nonionic surfactant having at least one olefin-based double bond in a hydrophobic group moiety is a nonionic surfactant selected from glycerol unsaturated fatty acid esters, polyglycerol unsaturated fatty acid esters, propylene glycol unsaturated fatty acid esters, sorbitan unsaturated fatty acid esters, polyoxyethylene sorbitan unsaturated fatty acid esters, polyoxyethylene unsaturated fatty acid esters, mannitol unsaturated fatty acid esters, pentaerythritol unsaturated fatty acid esters, sucrose unsaturated fatty acid esters, polyethylene glycol unsaturated alkyl ethers, and polypropylene glycol unsaturated alkyl ethers.

5. The ozonized surfactant according to claim 3, wherein the anionic surfactant having at least one olefin-based double bond in a hydrophobic group moiety is an anionic surfactant selected from unsaturated fatty acid salts, unsaturated aliphatic alcohol sulfate esters, α-sulfo unsaturated fatty acid esters, olefin sulfates, and olefin sulfonates.

6. The ozonized surfactant according to claim 3, wherein the cationic surfactant having at least one olefin-based double bond in a hydrophobic group moiety is a cationic surfactant selected from unsaturated aliphatic hydrocarbon group-monosubstituted trimethyl ammonium salts, unsaturated aliphatic hydrocarbon group-disubstituted dimethyl ammonium salts, unsaturated aliphatic hydrocarbon group-monosubstituted dimethyl benzyl ammonium salts, unsaturated aliphatic hydrocarbon group-monosubstituted amine organic acid salts, unsaturated aliphatic hydrocarbon group-disubstituted amine organic acid salts, and N-methyl bishydroxyethylamine unsaturated fatty acid ester hydrochlorides.

7. The ozonized surfactant according to claim 1, wherein the surfactant having at least one olefin-based double bond in a hydrophobic group moiety is a polyethylene glycol unsaturated alkyl ether.

8. The ozonized surfactant according to claim 1, wherein the surfactant having at least one olefin-based double bond in a hydrophobic group moiety is a polyoxyethylene sorbitan unsaturated fatty acid ester.

9. The ozonized surfactant according to any one of claims 4 to 8, wherein the unsaturated fatty acid has 8 to 24 carbon atoms.

10. The ozonized surfactant according to claim 5 or 6, wherein the unsaturated aliphatic hydrocarbon group has 8 to 24 carbon atoms.

11. The ozonized surfactant according to claim 8, wherein the polyoxyethylene sorbitan unsaturated fatty acid ester is polyoxyethylene sorbitan oleic acid ester.

12. A pharmaceutical drug which contains the ozonized surfactant according to any one of claims 1 to 11.

13. A bactericide which contains the ozonized surfactant according to any one of claims 1 to 11.

14. A disinfectant which contains the ozonized surfactant according to any one of claims 1 to 11.

15. A deodorant which contains the ozonized surfactant according to any one of claims 1 to 11.

16. A drug for athlete's foot treatment which contains the ozonized surfactant according to any one of claims 1 to 11.

17. An antifungal agent which contains the ozonized surfactant according to any one of claims 1 to 11.

18. A drug for treatment of decubital ulcers which contains the ozonized surfactant according to any one of claims 1 to 11.
